Europäisches Patentamt

European Patent Office

Office européen des brevets

(19)

(11) Veröffentlichungsnummer: **0 304 831 B1**

# (12) EUROPÄISCHE PATENTSCHRIFT

(45) Veröffentlichungstag der Patentschrift: **23.10.91**

(51) Int. Cl.5: **C07C 51/41, B01F 7/08**

(21) Anmeldenummer: **88113565.1**

(22) Anmeldetag: **20.08.88**

(54) **Verfahren zur kontinuierlichen Herstellung von Fettsäureseifen.**

(30) Priorität: **28.08.87 DE 3728811**

(43) Veröffentlichungstag der Anmeldung:
**01.03.89 Patentblatt 89/09**

(45) Bekanntmachung des Hinweises auf die
Patenterteilung:
**23.10.91 Patentblatt 91/43**

(84) Benannte Vertragsstaaten:
**BE CH DE ES FR GB IT LI NL SE**

(56) Entgegenhaltungen:
**DE-A- 1 493 310**
**GB-A- 989 330**
**GB-A- 2 103 616**
**US-A- 3 787 160**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Carduck, Franz-Josef, Dr.
Landstrasse 18
W-5657 Haan(DE)**
Erfinder: **Harth, Hubert, Dr.
Buchenstrasse 31
W-4000 Düsseldorf(DE)**
Erfinder: **Liebs, Harald
Solinger Strasse 30
W-5090 Leverkusen 1(DE)**

Rank Xerox (UK) Business Services

## Beschreibung

Die Erfindung betrifft ein Verfahren zur kontinuierlichen Herstellung von Fettsäureseifen durch Umsetzung von Fettsäuren mit festen Metallverbindungen, die der Reihe der Oxide, Hydroxide, Hydrogencarbonate und Carbonate von Metallen aus der von Erdalkalimetallen wie Magnesium, Calcium und Barium sowie von Blei, Aluminium, Zink, Cobalt, Eisen, Cadmium und Zirkonium gebildeten Gruppe ausgewählt sind, gegebenenfalls in Gegenwart von bis zu 10 Gew.-% Wasser, bezogen auf das Gesamtgewicht der Reaktanten.

Unter den erfindungsgemäß herzustellenden Fettsäureseifen sind Verbindungen zu verstehen, die formal durch Neutralisation von Fettsäuren mit stöchiometrischen Mengen von Metalloxiden, -hydroxiden, -hydrogencarbonaten und -carbonaten oder mit einem Überschuß dieser basischen Metallverbindungen gebildet werden. Metallseifen finden eine vielfältige Verwendung. Calciumseifen, insbesondere basische Calciumseifen, z.B. der Sojaspaltfettsäure und der Palmölfettsäure, werden z.B. in der Wiederkäuerfütterung eingesetzt; zweibasisches Bleistearat und komplexe Metall/Calciumstearate sind z.B. bekannte Additive für die PVC-Verarbeitung.

Metallseifen können batchweise hergestellt werden, indem man die geschmolzene Fettsäure mit einer Aufschlämmung oder Lösung von Metalloxiden oder Hydroxiden umsetzt, die entstehenden Seifen abfiltriert und trocknet. Diese Verfahrensweise ist jedoch aufwendig, da in hinreichend großer Verdünnung gearbeitet werden muß, um eine rührfähige Reaktionsmasse zu erhalten. Außerdem wird für die Trocknung der Seife zu einem rieselfähigen Produkt viel Energie verbraucht.

Einige basische Seifen, z.B. basische Bleistearate, können chargenweise in einem Schmelzprozeß hergestellt werden, indem das Metalloxid oder -hydroxid in die gerührte Schmelze der Fettsäure eingetragen wird. Bei diesen Verfahren kann es durch die lang andauernde thermische Belastung zu Produktverfärbungen kommen. Die basischen Seifen von Erdalkalimetallen sind nach diesem Verfahren überhaupt nicht herstellbar, da es sich hierbei um nicht-schmelzbare Massen handelt.

Andere basische Seifen, z.B. die Calciumseife der Palmölfettsäure, können batchweise in einem sogenannten Heizmischer (Fluidmischer) wie er in der PVC-Verarbeitung üblich ist, hergestellt werden, indem man in die vorgelegte geschmolzene Fettsäure das Metalloxid oder -hydroxid einträgt. Durch die Neutralisationswärme und die durch das Mischwerk eingetragene Energie steigt die Temperatur der Reaktionsmischung an, und das Reaktionswasser dampft teilweise aus. Nach Ausreaktion der Ausgangsstoffe bricht die Reaktionsmischung von einer zähviskosen in eine feste Struktur um. Diese Masse kann als Feststoff abgekühlt und zerkleinert werden. Beim Umbrechen von der zähviskosen in die feste Phase gerät der Mischer jedoch in starke Schwingungen, die an einem für die Produktion erforderlichen Reaktor von einigen 100 l Inhalt technisch nicht mehr zu beherrschen sind. Außerdem sind die vorgenannten Fluidmischer für ihren hohen Verbrauch an elektrischer Energie bekannt. Das Verfahren ist somit nur für die Herstellung kleiner Mengen für Labor und Technikum geeignet.

Weiterhin ist aus der EP-A 0 163 395 ein Verfahren zur Herstellung von Metallseifen bekannt, bei dem aus geschmolzenen Fettsäuren und basischen Metallverbindungen wie Calciumoxid ein pumpfähiges Gemisch hergestellt und auf einem Bandförderer zur Ausreaktion und zur Entfernung des enthaltenen bzw. bei der Reaktion gebildeten Wassers ausgebracht wird. Das erhaltene Reaktionsprodukt muß anschließend zerkleinert werden, was in Anbetracht der großen, zu bewältigenden Mengen und der zum Teil recht festen Konsistenz der ausreagierten Fettsäureseifen technologische Probleme aufwirft.

Es besteht somit ein Bedürfnis an einfachen, kostengünstigen und produktschonenden Verfahren zur Herstellung von Metallseifen der obengenannten Art. Überraschenderweise wurde nun gefunden, daß die genannten Metallseifen hergestellt werden, indem

a) die auf eine Temperatur von 20 bis 150° C erwärmten, geschmolzenen Fettsäuren durch mechanische Förderung in eine Rotationsbewegung versetzt und in eine Reaktionszone eines Rohrreaktors gefördert werden,

b) die Metallverbindungen durch mechanische Förderung mit einer Rotationsbewegung versehen, die derjenigen der Fettsäuren entgegengesetzt ist, und in der Nähe der Rotationsachse der Fettsäuren bzw. der Reaktionszone eingespeist werden,

c) das Gemisch aus Fettsäuren und Metallverbindungen durch die Reaktionszone des Rohrreaktors geführt und innerhalb von 0,5 bis 50 sec umgesetzt wird, wobei die mechanische Förderung des Gemisches im Sinne der Rotation der Fettsäuren erfolgt, und

d) die Fettsäureseifen am Ausgang der Reaktionszone entnommen werden.

Das den Rohrreaktor verlassende Reaktionsprodukt ist dünnflüssig bis zähviskos und verfestigt sich unter Abkühlen. Im Falle von basischen Calciumseifen wird das Reaktionsprodukt in Schuppenform erhalten, die leicht zu Pulver zerkleinert werden können. In Anbetracht der Tatsache, daß das Reaktionsprodukt den Rohrreaktor verläßt, nachdem es vollständig oder nahezu vollständig abreagiert hat, kann die Bildung schwer zu zerkleinernder kompakter Massen verhindert werden.

Die Reaktionsgeschwindigkeit und die Konsistenz des Reaktionsproduktes können durch Variation der Mischintensität in dem Rohrreaktor, der Reaktortemperatur und der Menge der zudosierten Fettsäuren eingestellt werden. Ausgehend von Fettsäuren mit einer Säurezahl von 180 bis 220 mg KOH/g erhält man Seifen mit einer Restsäurezahl um 1 mg KOH/g.

Als basische Komponenten werden Metalloxide, Metallhydroxide, Mischungen von Oxiden und Hydroxiden der gleichen oder unterschiedlicher Metalle sowie Hydrogencarbonate und Carbonate, gegebenenfalls in Mischung mit Oxiden und Hydroxiden, eingesetzt, und zwar vorzugsweise 1-3 Äquivalente der Metallverbindungen pro Mol Fettsäure. Es können sowohl reine Fettsäuren als auch Fettsäuremischungen, z.B. Sojaspaltfettsäure, Palmölfettsäure oder Stearin, eingesetzt werden. Verunreinigungen mit Triglyceriden und unverseifbaren Stoffen stören nicht. In einigen Fällen, insbesondere beim Einsatz von Metalloxiden und Carbonaten, kann es vorteilhaft sein, geringe Mengen Wasser in den Mischer zu dosieren, um die Reaktion in Gang zu setzen. Da zwangsläufig im Verlauf der Reaktion Wasser gebildet wird, beträgt die Obergrenze für den Wasserzusatz 10 Gew.-%.

Das Verfahren der Erfindung gestattet auch die Zudosierung von Zuschlagsstoffen, die an der Neutralisation nicht teilnehmen. So können bei der Herstellung basischer Calciumseifen für die Wiederkäuerfütterung weitere Futtermittel, z.B. Sojamehl, zugesetzt werden, wodurch ein Coating dieser Stoffe erreicht wird. Bei der Herstellung von basischem Bleistearat können z.B. verschiedene flüssige Ester, wie sie in den EP-A 0 184 128 und 0 184 129 beschrieben sind, zugesetzt werden, so daß fertige Compounds für die PVC-Extrusion entstehen.

Für das Verfahren der Erfindung besonders gut geeignet sind Durchlaufmischer, die mit schnell laufenden, schnecken-, vorzugsweise spiralförmigen Mischwerkzeugen in einem kleinen Volumen eine hohe Misch- und Scherintensität entfalten. Hierdurch wird einerseits die vollständige Reaktion der festen basischen Komponente mit den flüssigen Fettsäuren gewährleistet, während andererseits durch die geringe Verweilzeit im Mischer die Verfestigung der Reaktionsmasse außerhalb desselben stattfinden kann und die Reaktionsprodukte thermisch geschont werden.

Ein zur Durchführung des Verfahrens der Erfindung geeigneter Reaktor umfaßt einen mit schnell laufenden Förderelementen versehenen Rohrreaktor mit

    a) einer Förderzone und einer Reaktionszone, die hintereinander entlang der Rohrachse angeordnet sind,

    b) einem einseitig geschlossenen äußeren, die äußere Begrenzung der Förder- und Reaktionszone bildenden Rohr,

    c) einem vom geschlossenen Ende des äußeren Rohres sich koaxial über die Länge der Förderzone in das äußere Rohr erstreckenden inneren Rohr,

    d) einem in dem die Förderzone bildenden ringförmigen Raum zwischen dem äußeren und inneren Rohr und in der Reaktionszone angeordneten, sich koaxial drehenden äußeren Schneckenelement, und

    e) einem in dem inneren Rohr angeordneten, sich koaxial drehenden inneren Schneckenelement, dessen Drehrichtung derjenigen des äußeren Schneckenelementes entgegengesetzt ist.

Gemäß einer bevorzugten Ausführungsform der Erfindung ragt das reaktionszonenseitige Ende des inneren Schneckenelements um eine Länge entsprechend ca. einer Schneckensteigung aus dem das Schneckenelement umgebenden inneren Rohr heraus.

In der praktischen Durchführung wird die Fettsäure im geschmolzenen Zustand in den die Förderzone bildenden ringförmigen Raum zwischen dem äußeren und dem inneren Rohr eingespeist und mittels Spiralelementen in die Reaktionszone gefördert; die Zufuhr der festen Metallverbindungen erfolgt über das innere Rohr mittels einer rotierenden Spirale, deren Drehrichtung derjenigen der äußeren Spirale entgegengesetzt ist. Beim Zusammentreffen der mit entgegengesetztem Drehsinn rotierenden Komponenten entsteht die gewünschte homogene Reaktionsmischung.

Beim Austritt aus dem Mischer verdampft ein Teil des entstehenden Reaktionswassers. Für einige Anwendungsfälle, beispielsweise für basische Calciumseifen für die Wiederkäuerfütterung, kann das Reaktionswasser im Produkt verbleiben. In diesen Fällen kann die Reaktionsmasse auf Kühlbänder oder Kühlwalzen verteilt und durch Abkühlen verfestigt werden. Die festen basischen Seifen können dann auf die gewünschte Korngröße zerkleinert werden. Die Reaktionsmasse kann aber auch durch Versprühen in einem Kaltluftstrom verfestigt oder auf ein Wirbelbett aus bereits verfestigter basischer Seife aufgesprüht werden. Falls das Reaktionswasser im Produkt stört, z.B. für Anwendungen in der PVC-Verarbeitung, muß dieses aus der Reaktionsmasse entfernt werden. Dies kann z.B. dadurch geschehen, daß man die Reaktionsmasse in einen kontinuierlich betriebenen Kneter mit Austragsschnecke führt. Aus diesem Kneter wird das Wasser dampfförmig im Vakuum abgezogen. Durch Kühlung der Austragsschnecke erhält man eine Masse, die über Lochplatten zu Strängen geformt und nach Abkühlen zu Zylindergranulat gebrochen werden kann.

Anstelle eines Kneters können auch kontinuierlich arbeitende Hohlschnecken, Wärmetauscher, Entgasungsextruder und verwandte Einrichtungen eingesetzt werden.

Wie sich aus der vorstehenden Beschreibung ergibt, zeichnet sich die Durchführung des Verfahrens der Erfindung durch geringe Investitionskosten, geringen Energieverbrauch und besondere Einfachheit aus.

Das Verfahren der Erfindung wird im folgenden anhand eines bevorzugten Ausführungsbeispiels näher erläutert.

Beispiel.

Es wurde ein Rohrreaktor des vorstehend beschriebenen Typs mit den folgenden Daten eingesetzt:
Reaktorlänge (Summe von Förder- und Reaktionszone): 840 mm;
Gesamtlänge des inneren Rohres: 950 mm, davon ca. 200 mm innerhalb des äußeren Rohres;
Innendurchmesser des äußeren Rohres: 84 mm;
Innendurchmesser des inneren Rohres: 40 mm;
Außendurchmesser der äußeren Mischspirale: 75 mm;
Innendurchmesser der inneren Mischspirale: 55 mm;
Drehzahl der äußeren und der inneren Mischerspiralen jeweils 900 min$^{-1}$.

Der Mantel des Rohrreaktors wurde mit 60 $^\circ$C warmem Wasser beaufschlagt. Das innere Rohr des Reaktors wurde kontinuierlich mit 91 kg/h Ca-$(OH)_2$ beschickt. In das äußere Mischrohr wurden kontinuierlich 413 kg/h Sojaspaltfettsäure (Säurezahl = 186 mg KOH/g) von 56 $^\circ$C zudosiert; diese Bedingungen entsprachen einer Verweildauer der Reaktanten in der Reaktionszone von 1-5 sec.

Das aus dem Mischer austretende viskose Reaktionsprodukt hatte eine Restsäurezahl von 1,0 mg KOH/g und eine Austrittstemperatur von 65 $^\circ$C. Es wurde kontinuierlich auf ein mit Wasser gekühltes Stahlband (Breite 600 mm, Länge 10 m) verteilt und abgekühlt. Es wurden Schuppen erhalten, die mit einer sogenannten Reibe (Firma Alexander-Werke, Typ R300) zu Pulver zerkleinert wurden. Die so erhaltene basische Calciumseife der Sojaspaltfettsäure mit einem Calciumgehalt von 9,5 Gew.-% war als Zusatzstoff zu Futtermitteln für die Wiederkäuerfütterung gut geeignet.

**Patentansprüche**

1. Verfahren zur kontinuierlichen Herstellung von Fettsäureseifen durch Umsetzung von Fettsäuren mit festen Metallverbindungen, die aus der Reihe der Oxide, Hydroxide, Hydrogencarbonate und Carbonate von Metallen aus der von Erdalkalimetallen wie Magnesium, Calcium und Barium sowie von Blei, Aluminium, Zink, Cobalt, Eisen, Cadmium und Zirkonium gebildeten Gruppe ausgewählt sind, gegebenenfalls in Gegenwart von bis zu 10 Gew.-% Wasser, bezogen auf das Gesamtgewicht der Reaktanten, dadurch gekennzeichnet, daß

a) die auf eine Temperatur von 20 bis 150 $^\circ$C erwärmten, geschmolzenen Fettsäuren durch mechanische Förderung in eine Rotationsbewegung versetzt und in eine Reaktionszone eines Rohrreaktors gefördert werden,

b) die Metallverbindungen durch mechanische Förderung mit einer Rotationsbewegung versehen, die derjenigen der Fettsäuren entgegengesetzt ist, und in der Nähe der Rotationsachse der Fettsäuren bzw. der Reaktionszone eingespeist werden,

c) das Reaktionsgemisch aus Fettsäuren und Metallverbindungen durch die Reaktionszone des Rohrreaktors geführt und innerhalb von 0,5 bis 50 sec umgesetzt wird, wobei die mechanische Förderung des Gemisches im Sinne der Rotation der Fettsäuren erfolgt, und

d) die Fettsäureseifen am Ausgang der Reaktionszone entnommen werden.

2. Verfahren nach Anspruch 1, dadurch gekennzeichnet, daß man 1 bis 3 Äquivalente der Metallverbindungen pro Mol Fettsäure umsetzt.

3. Reaktor zur Durchführung des Verfahrens nach Anspruch 1 oder 2, gekennzeichnet durch einen mit schnell laufenden Förderelementen versehenen Rohrreaktor mit

a) einer Förderzone und einer Reaktionszone, die hintereinander entlang der Rohrachse angeordnet sind,

b) einem einseitig geschlossenen äußeren, die äußere Begrenzung der Förder- und Reaktionszone bildenden Rohr,

c) einem vom geschlossenen Ende des äußeren Rohres sich koaxial über die Länge der Förderzone in das äußere Rohr erstreckenden inneren Rohr,

d) einem in dem die Förderzone bildenden ringförmigen Raum zwischen dem äußeren und inneren Rohr und in der Reaktionszone angeordneten, sich koaxial drehenden äußeren Schneckenelement, und

e) einem in dem inneren Rohr angeordneten, sich koaxial drehenden inneren Schneckenelement, dessen Drehrichtung derjenigen des äußeren Schneckenelementes entgegengesetzt ist.

4. Reaktor nach Anspruch 3, dadurch gekennzeichnet, daß das reaktionszonenseitige Ende des inneren Schneckenelements um eine Länge entsprechend ca. einer Schneckensteigung aus dem das Schneckenelement umgebenden Rohr herausragt.

## Claims

1. A process for the continuous production of fatty acid soaps by reaction of fatty acids with solid metal compounds selected from the series of oxides, hydroxides, hydrogen carbonates and carbonates of metals from the group formed by alkaline earth metals, such as magnesium, calcium and barium, and by lead, aluminium, zinc, cobalt, iron, cadmium and zirconium, optionally in the presence of up to 10% by weight of water, based on the total weight of the reactants, characterized by
    a) subjecting the molten fatty acids heated to a temperature of 20 to 150°C to a rotational movement by mechanical transport and transporting them into the reaction zone of a tube reactor,
    b) subjecting the metal compounds by mechanical transport to a rotational movement in the opposite direction to the one of the fatty acids and introducing them in the vicinity of the axis of rotation of the fatty acids or rather of the reaction Zone,
    c) passing the mixture of fatty acids and metal compounds through the reaction zone of the tube reactor and reacting it in 0.5 to 50 seconds, the mixture being mechanically transported in the sense of the rotation of the fatty acids and
    d) removing the fatty acid soaps at the exit from the reaction zone.

2. A process as claimed in claim 1, characterized in that 1 to 3 equivalents of the metal compounds are reacted per mol fatty acid.

3. A reactor for carrying out the process claimed in claim 1 or 2, characterized by a tube reactor provided with highspeed feed elements and comprising
    a) a feed zone and a reaction zone arranged one behind the other along the tube axis,
    b) an outer tube closed at one end which forms the outer boundary of the feed zone and reaction zone,
    c) an inner tube extending coaxially from the closed end of the outer tube over the length of the feed zone into the outer tube,
    d) an outer, coaxially rotating screw element arranged in the annular space - forming the feed zone - between the outer tube and the inner tube and in the reaction zone and
    e) an inner, coaxially rotating screw element which is arranged in the inner tube and which rotates in the opposite direction to the outer screw element.

4. A reactor as claimed in claim 3, characterized in that the reaction zone end of the inner screw element projects from the tube surrounding the screw element by a distance corresponding to approximately one screw thread.

## Revendications

1. Procédé de fabrication en continu de savons d'acides gras, par mise en réaction d'acides gras avec des composés métalliques solides, qui sont sélectionnés dans la série des oxydes, hydroxydes, hydrogénocarbonates et carbonates de métaux faisant partie du groupe constitué par les métaux alcalino-terreux tels le magnésium, le calcium et le baryum ainsi que par le plomb, l'aluminium, le zinc, le cobalt, le fer, le cadmium et le zirconium, le cas échéant en présence d'une quantité d'eau allant jusqu'à 10 % en poids par rapport au poids total des réactifs, caractérisé en ce que
    a) les acides gras fondus, chauffés à une température située dans l'intervalle de 20 à 150°C, sont déplacés par manutention mécanique en un mouvement rotatif et transportés dans une zone réactionnelle d'un réacteur tubulaire,
    b) les composés métalliques sont dotés par manutention mécanique d'un mouvement de rotation, qui est opposé à celui des acides gras, et introduits à proximité de l'axe de rotation des acides gras ou de la zone réactionnelle,
    c) le mélange réactionnel des acides gras et des composés métalliques est acheminé par la zone réactionnelle du réacteur tubulaire où on le laisse réagir pendant 0,5 à 50 sec, la manutention mécanique du mélange se déroulant dans le sens de rotation des acides gras, et
    d) les savons d'acides gras sont récupérés à la sortie de la zone réactionnelle.

2. Procédé selon la revendication 1, caractérisé en ce que l'on fait réagir 1 à 3 équivalents des composés métalliques par mole d'acide gras.

3. Réacteur pour la réalisation du procédé selon la revendication 1 ou 2, caractérisé par un réacteur tubulaire doté d'éléments transporteurs à régime rapide avec

a) une zone de transport et me zone de réaction, qui sont aménagées à la suite l'une de l'autre, le long de l'axe du tube,

b) un tube externe fermé d'un côté, formant la limite avec l'extérieur de la zone de transport et de réaction,

c) un tube interne s'étendant dans le tube externe de manière coaxiale depuis l'extrémité fermée du tube externe sur toute la longueur de la zone de transport,

d) un élément à vis externe à rotation coaxiale, aménagé dans l'espace annulaire formant la zone de transport entre les tubes externe et interne et dans la zone réactionnelle, et

e) un élément à vis interne à rotation coaxiale, aménagé dans le tube interne, dont le sens de rotation est opposé à celui de l'élément à vis externe.

4. Réacteur selon la revendication 3, caractérisé en ce que l'extrémité de l'élément à vis interne du côté de la zone de réaction fait saillie d'une longueur correspondant à environ un pas de vis hors du tube qui entoure ledit élément à vis.